Europäisches Patentamt

European Patent Office

Office européen des brevets

(11) Publication number: **0 080 378**
A2

# EUROPEAN PATENT APPLICATION

(21) Application number: 82306254.2

(22) Date of filing: 24.11.82

(51) Int. Cl.³: **C 12 N 15/00**, C 12 P 13/22, C 12 N 1/20 // C12R1/07

(30) Priority: 24.11.81 JP 188089/81

(43) Date of publication of application: 01.06.83 Bulletin 83/22

(84) Designated Contracting States: **DE FR GB**

(71) Applicant: AJINOMOTO CO., INC., 5-8, Kyobashi 1-chome, Chuo-ku, Tokyo 104 (JP)

(72) Inventor: Tsuchida, Takayasu, No. 1730-13, Kamikurata-cho Totsuka-ku, Yokohama-shi Kanagawa-ken (JP)
Inventor: Kawashima, Nobuki, No. 2-20-8, Kannon Kawasaki-ku, Kawasaki-shi Kanagawa-ken (JP)
Inventor: Nakamori, Shigeru, No. 2153-187, Kamariya-cho Kanazawa-ku, Yokohama-shi Kanagawa-ken (JP)
Inventor: Enei, Hitoshi, No. 2-30-2, Ikego, Zushi-shi Kanagawa-ken (JP)
Inventor: Kurahashi, Osamu, No. 958, Kashimada Saiwai-ku, Kawasaki-shi Kanagawa-ken (JP)

(74) Representative: Bond, Bentley George et al, Haseltine Lake & Co. 28 Southampton Buildings Chancery Lane, London WC2A 1AT (GB)

(54) L-tryptophan-producing microorganisms.

(57) L-tryptophan producing microorganisms are constructed by incorporating, into a recipient strain of the genus Bacillus, a recombinant plasmid containing a DNA fragment controlling resistance to a tryptophan-antagonist, which DNA fragment is obtained from chromosal DNA of a mutant of the genus Bacillus resistant to the tryptophan-antagonist.

EP 0 080 378 A2

-1-

L-TRYPTOPHAN-PRODUCING MICROORGANISMS

This invention relates to microorganisms of the genus Bacillus constructed by a gene splicing technique, for producing L-tryptophan by fermentation.

There are two known processes for the microbiological production of L-tryptophan. One process is an enzymological process using indole and serine (or pyruvic acid and ammonia) as the starting materials. Another process is a fermentation process using carbohydrate as the carbon source, in which anthranilic acid or indole is sometimes used as an additional carbon source. The present invention concerns the latter fermentation process.

Examples of known microorganisms which produce L-tryptophan from carbohydrates by fermentation processes are a mutant of the genus Brevibacterium resistant to 5-methyltryptophan (US-A-3700539), a mutant of Corynebacterium resistant to tryptophan-analogue and phenylalanine-analogue and requiring phenylalanine and tyrosine for growth (US-A-3849251), a mutant of Bacillus resistant to 5-fluorotryptophan and requiring arginine, phenylalanine, lysine, leucine or purine for growth (JP-B-39517/1978), a mutant of Bacillus resistant to 5-fluorotryptophan (JP-A-148093/1976), and a mutant of Enterobacter (JP-A-57888/1976).

Examples of known microorganisms which produce L-tryptophan from carbohydrate and anthranilic acid by fermentation are wild strains of Aerobacter, Arthrobacter, Bacillus, Brevibacterium, Staphylococcus, Corynebacterium, Flavobacterium, Pseudomonas, Proteus, Serratia, Sarcina, Streptococcus, Xanthomonas, Candida, Saccharamyces, Zygosaccharomyces and Penicillium (JP-B-20711/1968), a mutant of Bacillus resistant to anthranilic acid and requiring anthranilic acid for growth (JP-B-29584/1972), a wild strain of Escherichia (FR-B-1207437) and a mutant of Bacillus resistant to 5-methyltryptophan (GB-A-1298499).

Examples of known microorganisms which produce L-tryptophan from carbohydrate and indole by fermentation are a wild strain of Candida (GB-A-1222904), a mutant of Escherichia requiring anthranilic acid (US-A-3293141), and a wild strain of Bacillus (US-A-3700558).

Other examples of known microorganisms which produce L-tryptophan from carbohydrate are those constructed by a gene splicing technique, namely Escherichia coli strains containing recombinant plasmid DNA (JP-A-71397/1982, JP-A-80398/1982 and Appl. Environ. Microbiol., 38, (2), 181-190, (1979)).

However, it has been difficult to use the above microorganisms in the commercial production of L-tryptophan because of the rather low L-tryptophan productivity of the microorganisms. A need therefore continues to exist for the development of novel microorganisms for the production of L-tryptophan in high yields.

According to the present invention, there is provided an L-tryptophan producing microorganism constructed by incorporating, into a recipient strain of the genus Bacillus, a recombinant plasmid DNA containing a DNA fragment controlling resistance to a trytophan-antagonist and obtained from chromosomal DNA of a mutant of the genus Bacillus resistant to the tryptophan-antagonist.

The present invention also provided an L-tryptophan producing microorganism constructed by incorporating a first recombinant plasmid into a first recipient strain of the genus Bacillus, said first recombinant plasmid containing a DNA fragment controlling resistance to an L-tryptophan-antagonist and obtained from a transformant of the genus Bacillus, which transformant has been produced by incorporating a second hybrid plasmid into a second recipient strain of the genus Bacillus, said second recombinant plasmid containing a DNA fragment controlling resistance to a tryptophan-antagonist and obtained from a mutant of the genus Bacillus resistant to a tryptophan-antagonist; said second recipient strain of the genus Bacillus being an L-tryptophan-requiring strain and said first recipient strain of the genus Bacillus being resistant to a tryptophan-antagonist.

The tryptophan-antagonists used in the present invention inhibit the growth of microorganisms of the genus Bacillus, but the inhibition is suppressed partly or completely when L-tryptophan coexists in the medium. Examples of tryptophan-antagonists are 4-fluorotryptophan, 5-fluorotryptophan, 6-fluorotryptophan, 7-fluorotryptophan, 4-methyltryptophan, 5-methyltryptophan, 6-methyltryptophan, 7-methyltryptophan, naphtylalanine, indorylacrylic acid, naphtylacrylic acid, β-(2-benzothienyl)alanine, styrylacetic acid and tryptazan.

Although any mutant of the genus Bacillus resistant to a tryptophan-antagonist can be used as the DNA-donor, mutants having high resistance are preferred. In many cases, better results can be obtained when mutants having a high productivity of L-tryptophan are used as the DNA-donor. The mutant resistant to a tryptophan-antagonist can be obtained by conventional mutation techniques, such as by exposing a parent strain of the genus Bacillus to 250 µg/ml of N-methyl-N'-nitro-N-nitrosoguanidine in a buffer solution and separating the colonies which appear on an agar medium containing an amount of tryptophan-antagonist inhibitive to the growth of the parent strain. Such DNA-donors naturally have a chromosomal DNA region controlling resistance to the tryptophan-antagonist.

The extraction of the chromosomal DNA can be carried out in a conventional manner, such as described in Bacteriol, 89, 1065, (1965).

As the vector DNA, plasmid DNAs which propagate in hosts of Bacillus can be used. Typical vector DNAs are pCT 127, pC 194, pC 221, pC 223 and pUB 112 (Proc. Natl. Acad. Sci. U.S.A., 74, 1680-1682 (1977)), pUB 110 (J.Bacteriol., 134, 318-329 (1978)), and pTP 4 and pTP 5 (Microbiol. Letters, 5, 55-59 (1978)), all of which are derived from plasmids of Staphylococcus, and pLS 15 and pLS 28 (J. Bacteriol., 131, 699-701 (1977)), pLS 13 (J. Bacteriol., 129, 1487-1494 (1977)), and pPL and pPL 2 (J. Bacteriol., 124, 484 (1975)), all of which are derived from plasmids of Bacillus.

The chromosomal DNA is digested with a restriction endonuclease by a known method (see, for example, Biochem. Biophys. Acta, 383, 457, (1975)). Various kinds of restriction endonucleases can be used, the degree of digestion being controlled by changing the reaction time.

The vector DNA is also cleaved with a restriction endonuclease. Suitable restriction endonucleases for each vector DNA are disclosed in the literature shown in the parenthesis above.

Recombination of the DNA to prepare the recombinant plasmid can be carried out by a ligation reaction with a ligase, or by incorporating, with terminal transferase, deoxyadenylic acid and thymidylic acid (or deoxyguanylic acid and deoxycytidylic acid) into the chromosomal DNA fragment and the cleaved vector DNA and by subjecting the modified chromosomal DNA fragment and the cleaved DNA to an annealing reaction.

The recombinant DNA thus obtained can be incorporated into the DNA-recipient, for example (a) by treating cells of the DNA-recipient with calcium chloride to increase the permeability (for example as is reported in respect of E. coli K-12 by M. Mandel and A. Higa in J. Mol. Biol., 53, 159 (1970)); or (b) by using, for the incorporation, cells of the DNA-recipient at a specific stage of growth when the cells become capable of incorporating plasmids (competent cell) (for example as is reported in respect of Bacillus subtilis by C.H. Duncan, G.A. Wilson and F.E. Young in Gene 1, 153 (1977)); or (c) by forming a protoplast or spheroplast of the DNA-recipient which easily incorporates plasmid DNA (for example as is known in respect of Bacillus subtilis, actinomycetes and yeast from S. Chang and S. N. Choen, Molec. Gen. Genet, 168, 111 (1979), M. J. Bibb, J. M. Ward and O. A. Hopwood, Nature, 274, 398 (1978) and A. Hinnen, J. B. Hicks and G. R. Fink, Proc. Natl. Acad. Sci., USA, 75, 1929 (1978)).

The recipients for the recombinant DNA are microorganisms of the genus Bacillus. It is convenient to use, as the DNA recipient, microorganisms sensitive to a tryptophan-analogue and requiring tryptophan for growth for the selection of a transformant containing a recombinant DNA containing a chromosomal DNA fragment controlling resistance to a tryptophan-antagonist. When the recombinant plasmid DNA containing a chromosomal DNA fragment controlling resistance to a tryptophan-antagonist is used for the transformation, after selection of the recombinant plasmid DNA using a host sensitive to a tryptophan-antagonist and requiring L-tryptophan for growth, microorganisms resistant to a tryptophan-antagonist and having no requirement of L-tryptophan for growth can be used as the DNA recipient.

The desired transformants are those which become resistant to the tryptophan-antagonist and are capable of producing L-tryptophan when microorganisms sensitive to the tryptophan-antagonist and requiring L-tryptophan for growth are used as the recipients. When microorganisms resistant to the tryptophan-antagonist and having no requirement of L-tryptophan for growth are used, the desired transformants are those which have the characteristics possessed by the vector DNA as the maker for selection.

The methods of cultivation of the L-tryptophan producing transformants thus obtained are conventional, and are similar to the methods for the cultivation of known L-tryptophan producing microorganisms. The aqueous culture medium employed may be a conventional one containing a carbon source, a nitrogen source, inorganic ions and, when required, minor organic nutrients such as vitamins and amino acids.

As to the carbon source, carbohydrates (such as glucose, sucrose, lactose and fructose) and raw materials containing such saccharides (such as starch hydrolysate, molasses and fruit juice) may be used. As well as carbohydrates, intermediate compounds for the production of L-tryptophan, such as anthranilic acid and indole, can be used as the carbon source. The concentration of the intermediate compound in the medium is preferably maintained at a low level, when it is used, to avoid inhibition of the growth of the transformant. Gaseous ammonia, aqueous ammonia, ammonium salts and other nitrogen containing materials can be used as the nitrogen source.

Cultivation of the microorganism is effected under aerobic conditions, the pH and the temperature of the aqueous culture medium preferably being adjusted to a suitable level previously determined, and may be continued until production of L-tryptophan ceases.

The invention will now be illustrated by the following Example.

## EXAMPLE

### (1) Extraction of chromosomal DNA

Bacillus subtilis AJ 11713 (FERM-BP 208), which requires L-arginine and L-leucine and is resistant to 5-fluorotryptophan, was cultured in 1 litre of "Bact Penassay Broth" (Difco) at 30°C for 2 hours with shaking, and cells in the exponential growth phase were harvested. Chromosomal DNA was extracted from the cells by a conventional phenol-method, whereby 4.1 mg of purified DNA were obtained.

### (2) Insertion of the chromosomal DNA fragment into a vector

As the vector, pUB 110 processing genetic information relating to kanamycin-resistance and neomycin-resistance was used. 10 μg of the chromosomal DNA obtained in step (1) and 5 μg of the vector DNA were digested separately with the endonuclease Eco RI at 37°C for 1 hour, and thereafter the two reaction mixtures were heated at 65°C for 10 minutes and mixed. The mixer solution was subjected to ligation reaction by a $T_4$ phage DNA-ligase in the presence of ATP and dithreitol at 10°C for 24 hours.

### (3) Genetic transformation with the plasmid containing the tryptophan producing gene

Bacillus subtilis AJ 11712, which requires L-arginine, L-leucine and L-tryptophan, was cultured in "Penassay Broth" (Difco) at 30°C overnight with shaking, thereafter cultured at 37°C for 4 hours with shaking in Medium-I (containing 0.5 g/dl of glucose, 0.2 g/dl of $(NH_4)_2SO_4$, 0.6 g/dl of $KH_2PO_4$, 1.4 g/dl of $K_2HPO_4$, 0.02 g/dl of $MgSO_4 . 7H_2O$, 0.1 g/dl of sodium citrate, 0.2 g/dl of yeast extract, 10 mg/dl of L-tryptophan, 25 mg/dl of L-arginine and 5 mg/dl of L-leucine), and further cultured, after the cultivation in Medium-I, at 37°C for 1.5 hours with shaking in Medium-II (containing 0.5 g/dl of glucose, 0.2 g/dl

of $(NH_4)_2SO_4$, 0.6 g/dl of $KH_2PO_4$, 1.4 g/dl of $K_2HPO_4$, 0.12 g/dl of $MgSO_4.7H_2O$, 0.1 g/dl of sodium citrate, 0.02 g/dl of yeast extract, 5 mg/dl of L-arginine and 0.5 mg/dl of L-leucine). Thus, competent cells having the ability of plasmid uptake were obtained (C. Anagnostopoulos, J. Spizizen, J. Bacteriol., 81, 741, (1961)).

To a suspension of the competent cells, there was added the hybrid plasmid containing the tryptophan producing gene obtained in step (2), and incubated at 37°C for 2 hours with shaking was carried out to complete the transformation reaction.

The cell-suspension was transferred onto a minimum medium prepared by adding 5 µg/ml of kanamycin, 10 mg/dl of L-leucine, 10 mg/dl of L-arginine and 2 g/dl of agar to a basal minimum medium having a pH of 7.2 and containing 0.6 g/dl of $KH_2PO_4$, 1.4 g/dl of $K_2HPO_4$. 0.2 g/dl of $(NH_4)_2SO_4$, 0.1 g/dl of sodium citrate, 0.02 g/dl of $MgSO_4.7H_2O$ and 0.5 g/dl of glucose, and cultured at 37°C. After 3 days cultivation, 3 colonies appeared on the agar medium, and were picked up for purification. The three transformants obtained were tryptophan prototroph and resistant to 5-fluorotryptophan, and all of the three transformants produced extracellular tryptophan.

Among the three transformants, Bacillus subtilis AJ 11714 (FERM-BP 209), which had highest productivity of L-tryptophan, was selected. The DNAs in Bacillus subtilis AJ 11714 were extracted from it by C.I. Kado's phenol method (J. Bac., 145, 3, 1365 (1981)). The plasmid DNA and chromosomal DNA were separated by agarose-gel electrophoresis, and the plasmid DNA obtained was purified by dialysis. The purified plasmid was incorporated into Bacillus subtilis AJ 11713, which produces L-tryptophan, in the manner described in step (3), whereby, as the desired transformant, kanamycin and 5-fluorotryptophan-resistant Bacillus subtilis AJ 11715 (FERM-BP 210) was obtained.

(5) Tryptophan production by the trypophan producers

The L-tryptophan productivity of Bacillus subtilis AJ 11713, Bacillus subtilis AJ 11714 and Bacillus subtilis AJ 11715 was tested as follows. Batches, each of 20ml, of a culture medium having a pH of 7.0 and containing, per decilitre, 8 g of glucose, 1 g of $NH_4Cl$, 0.2 g of KCl, 0.1 g of $KH_2PO_4$, 0.4 g of $MgSO_4.7H_2O$, 0.4 g of "casamino acid", 1 mg of $FeSO_4.4H_2O$, 1 mg of $MnSO_4.4H_2O$, 20 mg of L-arginine, 20 mg of L-leucine and 4 g of $CaCO_3$, were placed in 500 ml shaking flasks. In addition, 5 µg/ml of kanamycin were added to the medium for Bacillus subtilis AJ 11714 and Bacillus subtilis AJ 11715.

Cultivation was carried out at 30°C for 96 hours with shaking. After 48 hours of the cultivation, anthranilic acid was added to one batch of the medium so that the medium contained 0.5 g/dl thereof. Small amounts of indole were fed to another batch of the medium over a period extending from 48 hours to 96 hours, the total amount of indole fed being 0.5 g/dl.

The amounts of L-tryptophan in the supernatant of the resulting culture media (in mg/dl) were determined by microbiological-assay and are shown in the Table.

Table

| Microorganism | Additive | | |
| --- | --- | --- | --- |
| | Anthranilic acid | Indole | None |
| Bacillus subtilis AJ 11713 | 110 | 120 | 20 |
| Bacillus subtilis AJ 11714 | 140 | 155 | 70 |
| Bacillus subtilis AJ 11715 | 230 | 310 | 80 |

(6) Recovery of Bacillus subtilis AJ 11712

Bacillus subtilis AJ 11712 can be easily obtained by removing the plasmid from Bacillus subtilis AJ 11714 in a conventional manner such as follows. Bacillus subtilis AJ 11714 was cultured with shaking

0080378

in 4ml of CMG-2 medium having a pH of 7.0 and containing 0.5 g/dl of glucose, 1 g/dl of yeast extract, 1 g/dl of peptone and 0.5 g/dl of NaCl, placed in a 20 ml test tube. The temperature was adjusted at 30°C from initiation to 12 hours, and at 41°C from 12 hours to 36 hours. Cells in the resulting culture broth were collected, suspended in sterilized water, and spread on a CMG-2-agar-plate. The plate was then incubated at 30°C for one day, and was replicated onto a second CMG-2-agar-plate containing 10 μg/ml of kanamycin. The second CMG-2-agar-place was incubated at 30°C for 1 day. The strain which could not grow on the second CMG-2-agar-plate was separated as *Bacillus subtilis* AJ 11712.

RULE 28(1) INFORMATION

*Bacillus subtilis* AJ 11712, *Bacillus subtilis* AJ 11713, *Bacillus subtilis* AJ 11714 and *Bacillus subtilis* AJ 11715 have the taxonomic characteristics as disclosed in Proc. Sec. Int. Congr. Microbiol. (London, 1936), 245 (1937) and Nomencl. Comm. Intern. Soc. Microbiol., 28 (1937), and in additon have the characteristics as disclosed hereinabove.

*Bacillus subtilis* AJ 11713, *Bacillus subtilis* AJ 11714 and *Bacillus subtilis* AJ 11715 were deposited at the Fermentation Research Institute, Japan, on the 26th October 1981 under the accession numbers FERM-BP 208, FERM-BP 209 and FERM-BP 210, respectively, which depositions were converted to depositions under the Budapest Treaty on the 4th November 1982.

*Bacillus subtilis* AJ 11712 was deposited at the Fermentation Research Institute, Japan, on the 24th November, 1982 under the accession number FERM-BP 220.

0080378

CLAIMS

1. An L-tryptophan producing microorganism constructed by incorporating, into a recipient strain of the genus <u>Bacillus</u>, a recombinant plasmid DNA containing a DNA fragment controlling resistance to a trytophan-antagonist and obtained from chromosomal DNA of a mutant of the genus <u>Bacillus</u> resistant to the tryptophan-antagonist.

2. A microorganism as claimed in claim 1, wherein said tryptophan-antagonist is 5-fluorotryptophan.

3. A microorganism as claimed in claim 1 or 2, wherein said mutant and/or said recipient strain are of the species <u>Bacillus</u> <u>subtilis.</u>

4. A microorganism as claimed in any of claims 1 to 3, wherein said recipient strain requires L-tryptophan for growth.

5. A microorganism as claimed in any of claims 1 to 4, wherein said recipient strain is resistant to 5-fluorotryptophan or other tryptophan-antagonist.

6. An L-tryptophan producing microorganism constructed by incorporating a first recombinant plasmid into a first recipient strain of the genus <u>Bacillus</u>, said first recombinant plasmid containing a DNA fragment ccntrolling resistance to an L-tryptophan-antagonist and obtained from a transformant of the genus <u>Bacillus</u>, which transformant has been produced by incorporating a second hybrid plasmid into a second recipient strain of the genus <u>Bacillus</u>, said second recombinant plasmid containing a DNA fragment controlling resistance to a tryptophan-antagonist and obtained from a mutant of the genus <u>Bacillus</u> resistant to a tryptophan-antagonist; said second recipient strain of the genus <u>Bacillus</u> being an L-tryptophan-requiring strain and said first recipient strain of the genus <u>Bacillus</u> being resistant to a tryptophan-antagonist.

7. A microorganism as claimed in claim 6, wherein said mutant and/or said first recipient strain and/or

0080378

said second recipient strain are of the species
<u>Bacillus</u> <u>subtilis</u>.

8. <u>Bacillus</u> <u>subtilis</u> FERM-BP 209.

9. <u>Bacillus</u> <u>subtilis</u> FERM-BP 210.

10. A method of producing L-tryptophan, which
comprises aerobically culturing an L-tryptophan
producing microorganism as claimed in any of claims
1 to 9 in an aqueous culture medium.